# EUROPEAN PATENT APPLICATION

(11) **EP 3 628 257 A1**
(43) Date of publication of application: **01.04.2020**
(21) Application number: 19199943.2
(22) Date of filing: 26.09.2019
(51) Int. Cl.: A61B 18/16, A61B 17/29, A61B 18/14, A61B 17/00, A61B 17/02, A61B 17/42

(54) **ENERGY-BASED TISSUE SPECIMEN REMOVAL**

(30) Priority: 27.09.2018 US 201816143729
(71) Applicant: Covidien LP, Mansfield, MA 02048 (US)
(72) Inventor: DROCHNER, Thomas, E., Longmont, CO Colorado 80504 (US); ALLEN, James, D., Broomfield, CO Colorado 80020 (US); KINGSLEY, Dylan, R., Broomfield, CO Colorado 80020 (US); MANLEY, Prakash, Arvada, CO Colorado 80007 (US); MOUA, Tony, Broomfield, CO Colorado 80020 (US); ROBINSON, William, E., Boulder, CO Colorado 80301 (US); WHAM, Robert, H., Boulder, CO Colorado 80305 (US)
(74) Representative: Maschio, Antonio

(57) **Abstract**

A tissue removal system includes an electrosurgical generator including an active electrode port and a return electrode port, an active electrode device configured to connect to the active electrode port, and a return tissue clip configured to connect to the return electrode port. Another tissue removal system includes an electrosurgical generator including an active electrode port and a return electrode port, an active electrode device configured to connect to the active electrode port, and a return tissue harpoon configured to connect to the return electrode port. Another tissue removal system includes an active electrode device and a return electrode attachment configured to releasably engage the active electrode device.

## Description

### BACKGROUND

### Technical Field

The present disclosure relates to tissue specimen removal and, more particularly, to electrode configurations facilitating energy-based removal of a tissue specimen from an internal body cavity.

### Background of Related Art

In minimally-invasive surgical procedures, operations are carried out within an internal body cavity through small entrance openings in the body. The entrance openings may be natural passageways of the body or may be surgically created, for example, by making a small incision into which a cannula is inserted.

Minimally-invasive surgical procedures may be used for partial or total removal of tissue from an internal body cavity. However, the restricted access provided by minimally-invasive openings (natural passageways and/or surgically created openings) presents challenges with respect to maneuverability and visualization. The restricted access also presents challenges when large tissue specimens are required to be removed. As such, tissue specimens that are deemed too large for intact removal may be broken down into a plurality of smaller pieces to facilitate removal from the internal body cavity.

During such minimally-invasive surgical procedures, it is common that a cyst, tumor, or other affected tissue is required to be removed. In these and other procedures where cancerous tissue is required to be removed, removal of the tissue specimen(s) in an enclosed environment is highly desirable to inhibit seeding of cancer cells. Thus, with respect to breaking down large tissue specimens for removal through minimally-invasive openings, there is the added challenge of doing so within an enclosed environment.

### SUMMARY

As used herein, the term "distal" refers to the portion that is described which is further from a user, while the term "proximal" refers to the portion that is being described which is closer to a user. Further, any or all of the aspects described herein, to the extent consistent, may be used in conjunction with any or all of the other aspects described herein.

Provided in accordance with aspects of the present disclosure is a tissue removal system including an electrosurgical generator including an active electrode port and a return electrode port, an active electrode device configured to connect to the active electrode port, and a return tissue clip configured to connect to the return electrode port.

In an aspect of the present disclosure, the return tissue clip includes first and second arms movable about a hinge between a spaced-apart position and an approximated position to clamp tissue therebetween. At least one of the first or second arms includes an electrically-conductive portion. The return tissue clip further includes and a cable configured to connect the at least one electrically-conductive portion to the return electrode port.

In another aspect of the present disclosure, each of the first and second arms includes an electrically-conductive portion configured to connect to the return electrode port.

In still another aspect of the present disclosure, the electrosurgical generator is configured to monitor impedance between the electrically-conductive portions of the first and second arms and disable energy output from the active electrode port when the impedance is below a set point.

In yet another aspect of the present disclosure, at least one of the first or second arms includes tissue-engaging protrusions disposed thereon.

In still yet another aspect of the present disclosure, the return tissue clip includes a resistor circuit configured to override return electrode monitoring of the electrosurgical generator.

Another tissue removal system provided in accordance with aspects of the present disclosure includes an electrosurgical generator including an active electrode port and a return electrode port, an active electrode device configured to connect to the active electrode port, and a return tissue harpoon configured to connect to the return electrode port.

In an aspect of the present disclosure, the return tissue harpoon includes a shaft, at least one barb extending from the shaft, and a cable configured to connect to the return electrode port. The at least one barb includes an electrically-conductive portion.

In another aspect of the present disclosure, the at least one barb is selectively deployable from the shaft from a retracted position to a deployed position.

In still another aspect of the present disclosure, the return tissue harpoon includes first and second barbs each including an electrically-conductive portion configured to connect to the return electrode port.

In yet another aspect of the present disclosure, the electrosurgical generator is configured to monitor impedance between the first and second barbs and disable energy output from the active electrode port when the impedance is below a set point.

In still yet another aspect of the present disclosure, the return tissue harpoon includes a resistor circuit configured to override return electrode monitoring of the electrosurgical generator.

Another tissue removal system provided in accordance with aspects of the present disclosure includes an active electrode device and a return electrode attachment configured to releasably engage the active electrode device.

In an aspect of the present disclosure, the active electrode device includes a handle and an active electrode probe extending distally from the handle.

In another aspect of the present disclosure, the return electrode attachment includes a body, at least one attachment clamp extending from the body and configured to releasably engage the body of the return electrode attachment to the handle of the active electrode device, and a return electrode probe extending distally from the body of the return electrode attachment.

In still another aspect of the present disclosure, with the return electrode attachment engaged to the active electrode device, the return electrode probe extends in substantially parallel and spaced-apart relation relative to the active electrode probe.

In yet another aspect of the present disclosure, the return electrode attachment includes a suction lumen define therethrough to enable suction at a distal portion of the active electrode device when the return electrode attachment is engaged to the active electrode device.

In still yet another aspect of the present disclosure, the system further includes an electrosurgical generator including an active electrode port and a return electrode port. The active electrode device is configured to connect to the active electrode port, and the return electrode attachment is configured to connect to the return electrode port.

In another aspect of the present disclosure, the electrosurgical generator is configured to monitor impedance between first and second electrically-conductive portions of the return electrode attachment and disable energy output from the active electrode port when the impedance is below a set point.

In still another aspect of the present disclosure, the return electrode attachment includes a resistor circuit configured to override return electrode monitoring of the electrosurgical generator.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements and:
FIG. 1 is a transverse, cross-sectional view illustrating a tissue specimen removal system in accordance with the present disclosure in use removing a tissue specimen from an internal body cavity;
FIG. 2A is a transverse, cross-sectional view illustrating another tissue specimen removal system in accordance with the present disclosure, disposed in a retracted position, in use removing a tissue specimen from an internal body cavity;
FIG. 2B is a transverse, cross-sectional view illustrating the tissue specimen removal system of FIG. 2A, disposed in a deployed position, in use removing a tissue specimen from an internal body cavity;
FIG. 3A is a top view of a active electrode device including a return electrode device releasably coupled thereto in accordance with the present disclosure; and
FIG. 3B is a side view of the active electrode device of FIG. 3A including the return electrode device of FIG. 3A releasably coupled thereto.

### DETAILED DESCRIPTION

The present disclosure provides tissue removal systems including electrode configurations facilitating energy-based removal of a tissue specimen from an internal body cavity while maintaining the tissue specimen in an enclosed environment during break down and removal from the internal body cavity.

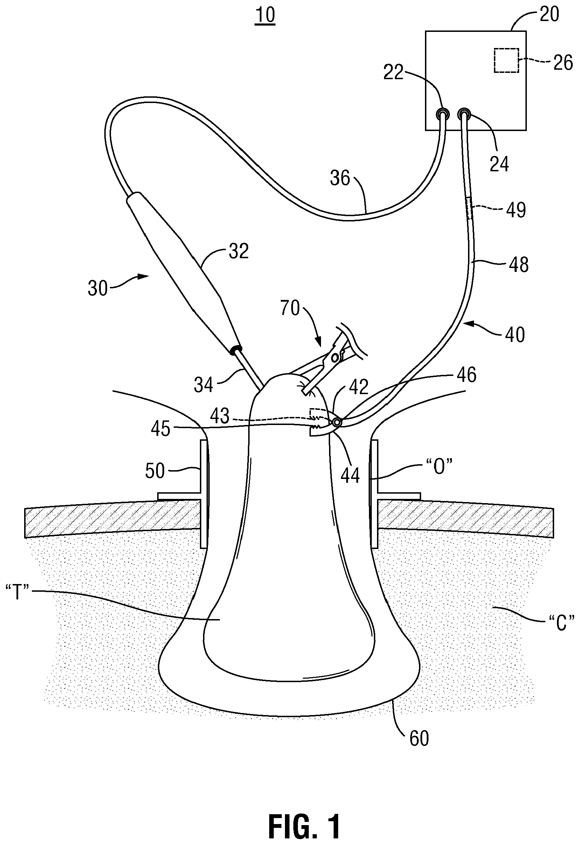

Turning to FIG. 1, a tissue removal system provided in accordance with the present disclosure is shown generally identified by reference numeral 10. System 10 includes an electrosurgical generator 20, an active electrode device, e.g., a monopolar surgical pencil 30, and a return electrode device, e.g., a return tissue clip 40. System 10 may further include an access port 50 configured for positioning within an opening "O" in tissue providing access to an internal body cavity "C," a specimen bag 60 configured to receive and retain a tissue specimen "T" therein to enable breakdown and removal of the tissue specimen "T" while the tissue specimen "T" remains isolated from the internal body cavity "C," and/or a tenaculum 70 to facilitate manipulation and extraction of the tissue specimen "T."

Electrosurgical generator 20 is configured to enable use in a monopolar mode and, thus, includes an active electrode port 22 and a return electrode port 24. Electrosurgical generator 20 may incorporate a Return Electrode Monitoring (REM) module 26 coupled to return electrode port 24 and configured to measure the impedance of the return electrode (which will vary based upon the contact area between tissue and the return electrode) and inhibit the output of energy via active electrode port 22 if the impedance drops below a set point (thus indicating insufficient contact area between tissue and the return electrode).

The active electrode device is configured as a monopolar surgical pencil 30, although other suitable active electrode devices are also contemplated. Monopolar surgical pencil 30 includes a handle 32, a probe 34 extending distally from handle 32, and a cable 36 extending proximally from handle 32. Handle 32 is configured to enable a user to grasp and manipulate monopolar surgical pencil 30 and may include one or more controls (not shown), e.g., switches, buttons, etc., to enable a user to control the application of energy from active electrode probe 34 to tissue. Probe 34 is at least partially formed from an electrically-conductive material to supply energy to tissue in contact therewith. Cable 36 terminates in a plug configured to engage active electrode port 22 of electrosurgical generator 20 to enable the supply of energy from electrosurgical generator 20 to probe 34, for application from probe 34 to tissue.

The return electrode device is configured as a return tissue clip 40 and serves as a return electrode pathway back to electrosurgical generator 20. Return tissue clip 40 includes first and second arms 42, 44 coupled to one another about a hinge 46, and a cable 48 coupled to arms 42, 44.

Arms 42, 44 are at least partially formed from an electrically-conductive material and, as noted above, are coupled to one another about hinge 46. More, specifically, the inner surfaces of arms may be electrically-conductive or include an electrically-conductive material disposed thereon while the outer surfaces of arms 42, 44 are electrically-insulative or include an electrically-insulative material disposed thereon. Arms 42, 44 are electrically coupled to cable 48 and may be coupled to the same electrical lead of cable 48 or first and second electrical leads of cable 48 that are electrically-isolated from one another. In embodiments, one or both arms 42, 44 may include one or more tissue-grasping protrusions, e.g., teeth 43, 45, and/or other complementary structures, at the distal ends thereof to facilitate grasping tissue therewith.

Hinge 46, as noted above, couples arms 42, 44 with one another and may be configured as a pivot-pin hinge, a living hinge, or other suitable hinge enabling movement of arms 42, 44 relative to one another between a spaced-apart position and an approximated position. Hinge 46 may be electrically-insulative or otherwise configured to electrically isolate arms 42, 44 from one another, e.g., in embodiments where arms 42, 44 are electrically isolated from one another, or may be electrically-conductive, e.g., in embodiments where arms 42, 44 are electrically coupled to one another.

Arms 42, 44, as noted above, are movable relative to one another about hinge 46 between the spaced-apart position and the approximated position for engaging return tissue clip 40 about tissue. To this end, hinge 46 may include a releasable locking mechanism (not shown), e.g., a shape-retention feature, ratchet engagement, spring mechanism, or other suitable locking feature, and/or arms 42, 44 may include complementary locking components (not shown) configured to releasably engage one another in order to lock arms 42, 44 in the approximated position about tissue.

Cable 48 is electrically coupled with arms 42, 44 and extends to a plug configured to engage return electrode port 24 of electrosurgical generator 20 to complete the circuit back to electrosurgical generator 20 from energy supplied to tissue from electrosurgical generator 20 via probe 34. In embodiments where arms 42, 44 are electrically-isolated from one another, cable 48 may include first and second leads (not shown) electrically-isolated from one another and coupled to the respective first and second arms 42, 44.

In use, with the tissue specimen "T" disposed within specimen bag 60 within the internal body cavity "C," return tissue clip 40 is maneuvered into position such that a portion of the tissue specimen "T" is disposed between arms 42, 44 of return tissue clip 40. Thereafter, arms 42, 44 are moved from the spaced-apart position to the approximated position and locked in the approximated position with tissue disposed therebetween, such that return tissue clip 40 is engaged about the tissue specimen "T" with arms 42, 44 in contact with the tissue specimen "T." Due to this engagement of return tissue clip 40 about the tissue specimen "T," contact between arms 42, 44 and the tissue specimen "T" is maintained even if the tissue specimen "T" is manipulated, e.g., using tenaculum 70.

With return tissue clip 40 engaged about the tissue specimen "T," tenaculum 70 may be utilized to grasp and manipulate the tissue specimen "T" to a desired position such that monopolar surgical pencil 30 may be maneuvered into position with probe 34 thereof contacting the tissue specimen "T." Thereafter, monopolar surgical pencil 30 may be activated to deliver energy to the tissue specimen "T" to cut the tissue specimen "T" into smaller pieces to facilitate removal from the internal body cavity "C." The energy supplied to the tissue specimen "T" from probe 34 of monopolar surgical pencil 30 is returned to electrosurgical generator 20 via the electrically-conductive arms 42, 44 of return tissue clip 40 which, as noted above, are disposed in contact with the tissue specimen "T" to complete the electrical circuit.

In embodiments where electrosurgical generator 20 includes REM module 26 and REM is activated, cable 48 may include a resistor circuit 49 configured to provide the requisite impedance to REM module 26 such that REM module 26 does not inhibit the output of energy via active electrode port 22. Alternatively, in embodiments where first and second arms 42, 44 are electrically-isolated from one another, REM module 26 may measure the impedance therebetween and inhibit the output of energy via active electrode port 22 if the impedance drops below the set point.

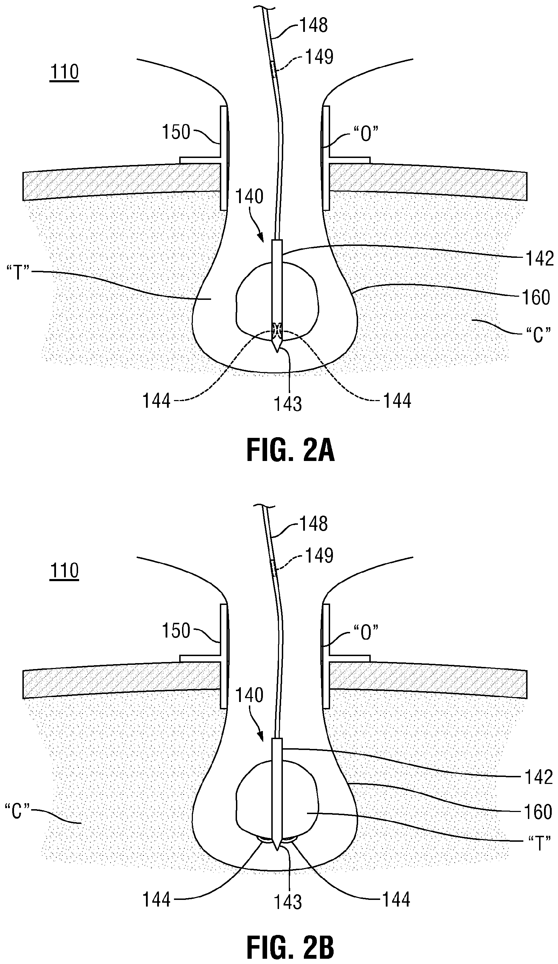

Turning now to FIGS. 2A and 2B, another tissue removal system provided in accordance with the present disclosure is shown generally identified by reference numeral 110. System 110 includes an electrosurgical generator (not shown, similar to electrosurgical generator 20 (FIG. 1)), an active electrode device, e.g., a monopolar surgical pencil (not shown, similar to monopolar surgical pencil 30 (FIG. 1)), and a return electrode device, e.g., a return tissue harpoon 140. System 110 may further include an access port 150 configured for positioning within an opening "O" in tissue providing access to an internal body cavity "C," a specimen bag 160 configured to receive and retain a tissue specimen "T" therein to enable breakdown and removal of the tissue specimen "T" while the tissue specimen "T" remains isolated from the internal body cavity "C," and/or a tenaculum (not shown, similar to tenaculum 70 (FIG. 1)) to facilitate manipulation and extraction of the tissue specimen "T."

System 110 is configured and functions similar to system 10 (FIG. 1) except for the return electrode device. Accordingly, only return tissue harpoon 140 is described in detail below to avoid unnecessary repetition.

Return tissue harpoon 140 includes a shaft 142, a plurality of tissue barbs 144, e.g., two tissue barbs 144, and a cable 148 extending from the proximal end portion of shaft 142. Shaft 142 may be formed from or coated with an electrically insulative material and may define a tapered distal end portion 143 to facilitate penetration through the tissue specimen "T." Tissue barbs 144 are selectively deployable from shaft 142 towards the distal end thereof such that, in the retracted position (FIG. 2A), insertion of shaft 142 into the tissue specimen "T" is facilitated and, in the deployed position (FIG. 2B), withdrawal of shaft 142 from the tissue specimen "T" is inhibited. Alternatively, tissue barbs 144 may permanently extend from shaft 142 in a proximally-facing direction to permit distal insertion of return tissue harpoon 140 into the tissue specimen "T" and inhibit proximal retraction of return tissue harpoon 140 from the tissue specimen "T."

Barbs 144 are at least partially formed from an electrically-conductive material. More specifically, barbs 144 are electrically coupled to cable 148 and may be coupled to the same electrical lead of cable 148 (in embodiments where barbs 144 are electrically coupled to one another) or different barbs 144 may be electrically isolated from one another and electrically coupled to first and second electrical leads of cable 148 that are electrically-isolated from one another.

Cable 148 is electrically coupled with barbs 144 and extends to a plug (not shown) configured to engage the return electrode port of the electrosurgical generator (not shown) to complete the circuit back to the electrosurgical generator from energy supplied to tissue from electrosurgical generator via the active electrode device (not shown). In embodiments where different barbs 144 are electrically-isolated from one another, as noted above, cable 148 may include first and second leads (not shown) electrically-isolated from one another and coupled to the respective barbs 144.

In use, with the tissue specimen "T" disposed within specimen bag 160 within the internal body cavity "C," return tissue harpoon 140 is inserted through the tissue specimen "T" sufficiently such that distal end portion 143 of shaft 142 is lodged within or extends through the tissue specimen "T." Thereafter, barbs 144 are deployed from shaft 142 to extend outwardly into contact with the tissue specimen "T," thereby maintaining return tissue harpoon 140 in engagement with the tissue specimen "T."

With return tissue harpoon 140 engaged with the tissue specimen "T" and barbs 144 in contact with the tissue specimen "T," the tissue specimen "T" may be manipulated using return tissue harpoon 140, e.g., the proximal end of shaft 142 or cable 148, and/or using the tenaculum (not shown) to a desired position such that the active electrode device (not shown) may be maneuvered into contact with the tissue specimen "T." Thereafter, the active electrode device may be activated to deliver energy to the tissue specimen "T" to cut the tissue specimen "T" into smaller pieces to facilitate removal from the internal body cavity "C." The energy supplied to the tissue specimen "T" from the active electrode device is returned to the electrosurgical generator via barbs 144 of return tissue harpoon 140 which, as noted above, are disposed in contact with the tissue specimen "T" to complete the electrical circuit.

In embodiments where the electrosurgical generator includes a REM module and REM is activated, cable 148 may include a resistor circuit 149 configured to provide the requisite impedance to the REM module such that the REM module does not inhibit the output of energy via the active electrode port. Alternatively, in embodiments where different barbs 144 are electrically-isolated from one another, the REM module may measure the impedance therebetween and inhibit the output of energy via the active electrode port if the impedance drops below the set point.

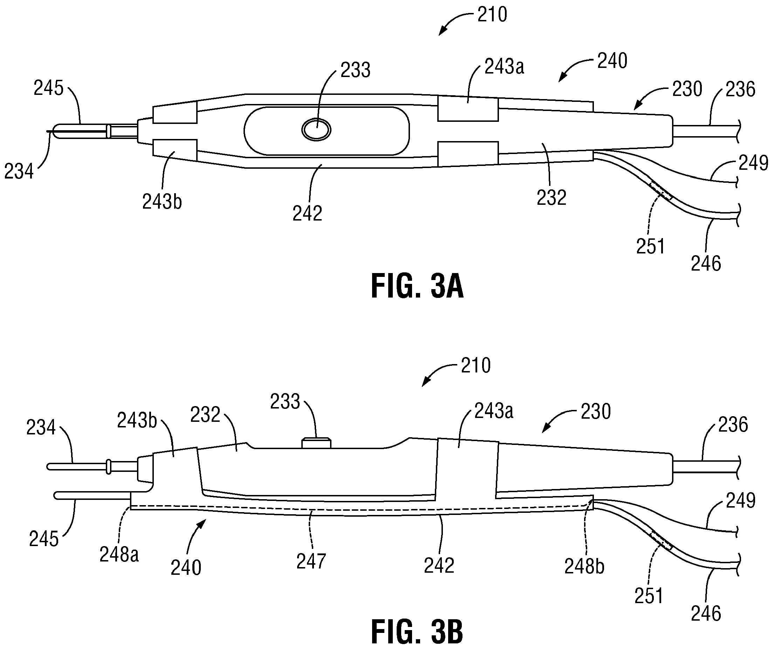

With reference to FIGS. 3A and 3B, another tissue removal system provided in accordance with the present disclosure is shown generally identified by reference numeral 210. System 210 includes an electrosurgical generator (not shown, similar to electrosurgical generator 20 (FIG. 1)), an active electrode device, e.g., a monopolar surgical pencil 230, and a return electrode device, e.g., a return and suction attachment 240. System 210 may further include an access port (not shown, similar to access port 50 (FIG. 1)) configured for positioning within an opening in tissue providing access to an internal body cavity, a specimen bag (not shown, similar to specimen bag 60 (FIG. 1)) configured to receive and retain a tissue specimen therein to enable breakdown and removal of the tissue specimen while the tissue specimen remains isolated from the internal body cavity, and/or a tenaculum (not shown, similar to tenaculum 70 (FIG. 1)) to facilitate manipulation and extraction of the tissue specimen.

System 210 is configured and functions similar to system 10 (FIG. 1) except for the return electrode device. Accordingly, only return and suction attachment 240 and, to the extent necessary, monopolar surgical pencil 230 are described in detail below to avoid unnecessary repetition.

The active electrode device is configured as a monopolar surgical pencil 230, although other suitable active electrode devices are also contemplated. Monopolar surgical pencil 230 includes a handle 232 having an activation button 233 disposed thereon, a probe 234 extending distally from handle 232, and a cable 236 extending proximally from handle 232 and configured to engage the active electrode port of the electrosurgical generator to enable the supply of energy from the electrosurgical generator to probe 234, for application from probe 234 to tissue.

The return electrode device is configured as return and suction attachment 240 that is releasably engagable with monopolar surgical pencil 230. Return and suction attachment 240 includes a body 242, one or more attachment clamps, e.g., proximal and distal attachment clamps 243a, 243b, respectively, a return electrode probe 245, a cable 246, a suction lumen 247 having an open distal end 248a, and a suction tube 249 disposed in communication with a proximal end 248b of suction lumen 247. Return and suction attachment 240 may further include one or more controls (not shown), e.g., switches, buttons, etc., to enable a user to selectively activate or deactivate suction. Suction tube 249 is adapted to connect to a suitable suction source (not shown) which may additionally or alternatively include the one or more controls to enable activation and deactivation of suction.

Body 242 is configured to extend along handle 232 of monopolar surgical pencil 230 while proximal and distal attachment clamps 243a, 243b extend about at least a portion of handle 232 to releasably engage return and suction attachment 240 about monopolar surgical pencil 230. More specifically, proximal and distal attachment clamps 243a, 243b define generally C-shaped configurations and are configured to flex to receive handle 232 therebetween and resiliently return to capture handle 232 therein, thereby engaging monopolar surgical pencil 230. Proximal and distal attachment clamps 243a, 243b are sufficiently spaced so as to avoid interfering with activation button 233 of monopolar surgical pencil 230.

With return and suction attachment 240 engaged about monopolar surgical pencil 230, return electrode probe 245 extends distally from body 242 in substantially parallel and space-apart relation (wherein "substantially" accounts for material, manufacturing, and attachment tolerances) relative to probe 234 of monopolar surgical pencil 230. Return electrode probe 245 may define a spatula-shaped configuration, a tubular configuration, or any other suitable configuration and may be formed from, coated within, incorporate, or otherwise include one or more electrically-conductive portions to serve as a return electrode. Return electrode probe 245 may be coupled to a single electrical lead of cable 246 (in embodiments where a single electrically-conductive portion is provided) or different electrically-conductive portions of return electrode probe 245 may be electrically isolated from one another and electrically coupled to first and second electrical leads of cable 246 that are electrically-isolated from one another.

Suction lumen 247 extends through body 242 of return and suction attachment 240 and defines an open distal end 248a positioned adjacent return electrode probe 245 to enable the evacuation of smoke, fluids, and/or debris, etc. from the surgical site. As noted above, suction lumen 247 extends from open distal end 248a proximally through body 242 to proximal end 248b, wherein suction lumen 247 communicates with suction tube 249 to enable any smoke, fluids, and/or debris, etc. evacuated from the surgical site to be deposited in a suitable collection reservoir (not shown).

Cable 246 is electrically coupled with the one or more electrically-conductive portions of return electrode probe 245 and extends to a plug (not shown) configured to engage the return electrode port of the electrosurgical generator (not shown) to complete the circuit back to the electrosurgical generator from energy supplied to tissue from electrosurgical generator via monopolar surgical pencil 230. In embodiments where different electrically-conductive are provided and electrically-isolated from one another, as noted above, cable 246 may include first and second leads (not shown) electrically-isolated from one another and coupled to the respective electrically-conductive portions.

In use, with, for example, a tissue specimen disposed within a specimen bag within an internal body cavity, the tissue specimen may be manipulated using a tenaculum (not shown) to a desired position. Thereafter, monopolar surgical pencil 230, having return and suction attachment 240 engaged thereon, may be maneuvered such that both probe 234 of monopolar surgical pencil 230 and return electrode probe 245 are disposed in contact with the tissue specimen. Once this contact is achieved, monopolar surgical pencil 230 may be activated, e.g., using activation button 233, to deliver energy to the tissue specimen to cut the tissue specimen into smaller pieces to facilitate removal from the internal body cavity. The energy supplied to the tissue specimen from monopolar surgical pencil 230 is returned to the electrosurgical generator via return electrode probe 245 of return and suction attachment 240 which, as noted above, is disposed in contact with the tissue specimen to complete the electrical circuit. Before, during, and/or after energy application, suction may be activated to enable any smoke, fluids, and/or debris, etc. to be evacuated from the surgical site.

In embodiments where the electrosurgical generator includes a REM module and REM is activated, cable 246 may include a resistor circuit 251 configured to provide the requisite impedance to the REM module such that the REM module does not inhibit the output of energy via the active electrode port. Alternatively, in embodiments where different electrically-conductive portions of return electrode probe 245 are electrically-isolated from one another, the REM module may measure the impedance therebetween and inhibit the output of energy via the active electrode port if the impedance drops below the set point.

From the foregoing and with reference to the various drawings, those skilled in the art will appreciate that certain modifications can be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A tissue removal system, comprising:
   an electrosurgical generator including an active electrode port and a return electrode port;
   an active electrode device configured to connect to the active electrode port; and
   at least one of a return tissue clip or a return tissue harpoon configured to connect to the return electrode port.
2. The tissue removal system according to paragraph 1, wherein the return tissue clip includes first and second arms movable about a hinge between a spaced-apart position and an approximated position to clamp tissue therebetween, at least one of the first or second arms including an electrically-conductive portion, and wherein the return tissue clip further includes and a cable configured to connect the at least one electrically-conductive portion to the return electrode port.
3. The tissue removal system according to paragraph 2, wherein each of the first and second arms includes an electrically-conductive portion configured to connect to the return electrode port.
4. The tissue removal system according to paragraph 3, wherein the electrosurgical generator is configured to monitor impedance between the electrically-conductive portions of the first and second arms and disable energy output from the active electrode port when the impedance is below a set point.
5. The tissue removal system according to paragraph 1, wherein the at least one of the return tissue clip or the return tissue harpoon includes a resistor circuit configured to override return electrode monitoring of the electrosurgical generator.
6. The tissue removal system according to paragraph 1, wherein the return tissue harpoon includes a shaft, at least one barb extending from the shaft, and a cable configured to connect to the return electrode port, the at least one barb including an electrically-conductive portion.
7. The tissue removal system according to paragraph 6, wherein the return tissue harpoon includes first and second barbs each including an electrically-conductive portion configured to connect to the return electrode port.
8. The tissue removal system according to paragraph 7, wherein the electrosurgical generator is configured to monitor impedance between the first and second barbs and disable energy output from the active electrode port when the impedance is below a set point.
9. A tissue removal system, comprising:
   an active electrode device; and
   a return electrode attachment configured to releasably engage the active electrode device.
10. The tissue removal system according to paragraph 9, wherein the active electrode device includes a handle and an active electrode probe extending distally from the handle.
11. The tissue removal system according to paragraph 10, wherein the return electrode attachment includes a body, at least one attachment clamp extending from the body and configured to releasably engage the body of the return electrode device to the handle of the active electrode device, and a return electrode probe extending distally from the body of the return electrode attachment.
12. The tissue removal system according to paragraph 9, wherein the return electrode attachment includes a suction lumen define therethrough to enable suction at a distal portion of the active electrode device when the return electrode attachment is engaged to the active electrode device.
13. The tissue removal system according to paragraph 9, further comprising:
   an electrosurgical generator including an active electrode port and a return electrode port,
   wherein the active electrode device is configured to connect to the active electrode port, and wherein the return electrode attachment is configured to connect to the return electrode port.
14. The tissue removal system according to paragraph 13, wherein the electrosurgical generator is configured to monitor impedance between first and second electrically-conductive portions of the return electrode attachment and disable energy output from the active electrode port when the impedance is below a set point.
15. The tissue removal system according to paragraph 14, wherein the return electrode attachment includes a resistor circuit configured to override return electrode monitoring of the electrosurgical generator.

## Claims

1. A tissue removal system, comprising:
an electrosurgical generator including an active electrode port and a return electrode port;
an active electrode device configured to connect to the active electrode port; and
at least one of a return tissue clip or a return tissue harpoon configured to connect to the return electrode port.

2. The tissue removal system according to claim 1, wherein the return tissue clip includes first and second arms movable about a hinge between a spaced-apart position and an approximated position to clamp tissue therebetween, at least one of the first or second arms including an electrically-conductive portion, and wherein the return tissue clip further includes and a cable configured to connect the at least one electrically-conductive portion to the return electrode port.

3. The tissue removal system according to claim 2, wherein each of the first and second arms includes an electrically-conductive portion configured to connect to the return electrode port.

4. The tissue removal system according to claim 3, wherein the electrosurgical generator is configured to monitor impedance between the electrically-conductive portions of the first and second arms and disable energy output from the active electrode port when the impedance is below a set point.

5. The tissue removal system according to any preceding claim, wherein the at least one of the return tissue clip or the return tissue harpoon includes a resistor circuit configured to override return electrode monitoring of the electrosurgical generator.

6. The tissue removal system according to any preceding claim, wherein the return tissue harpoon includes a shaft, at least one barb extending from the shaft, and a cable configured to connect to the return electrode port, the at least one barb including an electrically-conductive portion.

7. The tissue removal system according to claim 6, wherein the return tissue harpoon includes first and second barbs each including an electrically-conductive portion configured to connect to the return electrode port.

8. The tissue removal system according to claim 7, wherein the electrosurgical generator is configured to monitor impedance between the first and second barbs and disable energy output from the active electrode port when the impedance is below a set point.

9. A tissue removal system, comprising:
an active electrode device; and
a return electrode attachment configured to releasably engage the active electrode device.

10. The tissue removal system according to claim 9, wherein the active electrode device includes a handle and an active electrode probe extending distally from the handle.

11. The tissue removal system according to claim 10, wherein the return electrode attachment includes a body, at least one attachment clamp extending from the body and configured to releasably engage the body of the return electrode device to the handle of the active electrode device, and a return electrode probe extending distally from the body of the return electrode attachment.

12. The tissue removal system according to claim 9, wherein the return electrode attachment includes a suction lumen define therethrough to enable suction at a distal portion of the active electrode device when the return electrode attachment is engaged to the active electrode device.

13. The tissue removal system according to any one of claims 9 to 12, further comprising:
an electrosurgical generator including an active electrode port and a return electrode port,
wherein the active electrode device is configured to connect to the active electrode port, and wherein the return electrode attachment is configured to connect to the return electrode port.

14. The tissue removal system according to claim 13, wherein the electrosurgical generator is configured to monitor impedance between first and second electrically-conductive portions of the return electrode attachment and disable energy output from the active electrode port when the impedance is below a set point.

15. The tissue removal system according to claim 14, wherein the return electrode attachment includes a resistor circuit configured to override return electrode monitoring of the electrosurgical generator.
